Europäisches Patentamt

European Patent Office

Office européen des brevets

(11) Publication number: **0 207 138**
**B1**

(12) **EUROPEAN PATENT SPECIFICATION**

(45) Date of publication of patent specification: **29.08.90**

(21) Application number: **86900526.4**

(22) Date of filing: **17.12.85**

(86) International application number:
**PCT/US85/02513**

(87) International publication number:
**WO 86/03679 03.07.86 Gazette 86/14**

(51) Int. Cl.⁵: **A 61 K 33/44**

(54) PROCESS TO PRODUCE A STABLE COAL TAR-CONTAINING SHAMPOO.

(30) Priority: **18.12.84 US 682912**

(43) Date of publication of application:
**07.01.87 Bulletin 87/02**

(45) Publication of the grant of the patent:
**29.08.90 Bulletin 90/35**

(84) Designated Contracting States:
**AT BE CH DE FR GB IT LI LU NL SE**

(56) References cited:
**FR-A-2 515 034**

**HANDBOOK OF NON PREDESCRIPTION DRUGS, 5th ed., 1977, American Pharmaceutical Association, Washington, DC (US); pp. 340-341 BALSAM ET21 - "Cosmetics science and technology", 2nd ed., vol. 2, 1972, Wiley Interscience; pp. 73-82, 91, 100-101**

(73) Proprietor: **NEUTROGENA CORPORATION**
**5755 West 96th Street**
**Los Angeles California 90045 (US)**

(72) Inventor: **FONG, John**
**853 Temple Terrace**
**Los Angeles, CA 90042 (US)**
Inventor: **NOUKARIKIAN, Vartan**
**918 East Acacia Apt. B**
**Glendale, CA 91205 (US)**
Inventor: **JUNGERMANN, Eric**
**2323 N. Central Avenue**
**Phoenix, AZ 85004 (US)**

(74) Representative: **Coxon, Philip et al**
**Eric Potter & Clarkson St. Mary's Court St. Mary's Gate**
**Nottingham NG1 1LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Courier Press, Leamington Spa, England.

# EP 0 207 138 B1

**Description**

This invention relates to a novel process for the production of a unique coal tar shampoo and more particularly to a process which enables the consistant replication of a coal tar shampoo at a given pH which has a clear, light color and is capable of maintaining its clarity and color as well as its preselected viscosity for periods well beyond the shelf-life heretofore accepted as inevitable.

Shampoo systems containing coal tar have heretofore been available for therapeutic use to control specified dermatological conditions such as seborrhea, psoriasis, and subacute and chronic eczematous conditions of the scalp. While such products have been well accepted as capable of achieving their stated therapeutic goals, the inability of such products to maintain a stable viscosity, color or clarity over time, i.e., an economically viable shelf life, has caused great waste and rendered the product extremely costly. Additionally, the desired pH for such preparations was exceedingly difficult to reproduce with consistancy which further increased unit costs. Another major problem of these prior products was the instability of their viscosity which has fallen to as low as 1700 cps upon aging, the desired viscosity for such products being in the range of 5,000—10,000 cps. Furthermore, aging and exposure to heat heretofore caused both the color and clarity of the prior art products to deteriorate to an appearance which was totally unacceptable to the consuming public. En toto, these problems caused such products to suffer an inordinately high number of returns.

The successful resolution of the aforementioned problems found in prior art coal tar shampoo has far more significance than economic impact. Because of the aesthetically displeasing nature of low viscosity, darkened and hazy product, consumers often do not use the product as consistently as they should. In order for the consumer to realize improvements in his/her scalp conditions, he/she must use the product on a continuous basis. In contrast, a coal tar shampoo having superior viscosity, color, and clarity will promote good regimental compliance and therefore is therapeutically beneficial to the user. Thus, eliminating the problems which plagued prior coal tar shampoos will provide a product whose qualities make them pleasant to use and encourages better patient compliance.

The health care industry previously addressed such shelf life problems in conventional shampoos with some degree of success. For instance, the stability of viscosity in such formulations was resolved by the additions of gums and polymers while pH reproducibility was realized by the use of selected buffers. However, these traditional approaches proved unworkable in attempting to resolve the stated problems in systems containing crude coal tar as an active ingredient. Nor has any technique heretofore been devised which resolves the problems which arise with the color and clarity of coal tar-containing shampoo systems on aging.

The present invention is predicated upon the discovery of a novel and unique process for producing a clear, light colored coal tar-containing shampoo which is especially stable with respect to viscosity, color and clarity over time whereby an economically viable shelf-life is obtained and the great waste heretofore associated with such a product is eliminated. Additionally, this new process results in a product with a more consistent pH. Moreover, the product produced by the novel process of this invention retains all of the traditional therapeutic values heretofore associated with Coal Tar shampoos, that is, it is effective to control symptoms of seborrhea, dandruff, psoriasis and subacute and chronic eczematous conditions of the scalp.

More particularly, the unique process hereof is predicated upon our discovery that unique and unexpected benefits are obtainable when the ratio of fatty acid diethanolamide ("Fatty Acid DEA") to cocamidopropyl betaine ("CAPB") in the coal tar shampoo formulation is strictly limited to the range of 1.3—2.6:1, the preservative and fragrance are deliberately preblended into the Fatty Acid DEA phase without heat before introduction into the master batch, and the coal tar shampoo is neutralized at the beginning of the process cycle rather than at the end as has been heretofore the common practice.

Accordingly, a principal object of the present invention is to provide a new and unique process for producing an improved coal tar shampoo which results in a clear, light colored product capable on aging of maintaining its clarity, color, and viscosity.

Another object of the present invention is to provide a new and unique process for producing an improved coal tar shampoo in which the ratio of fatty acid diethanolamide to cocamidopropyl betaine is maintained in the range between 1.3:1 and 2.6:1.

A further object of the present invention is to provide a new and improved process for producing coal tar shampoo in which the fragrance and the preservative are preblended into the Fatty Acid DEA phase without heat before the Fatty Acid DEA blend is admixed into the master batch.

Another object of the present invention is to provide a new and improved process for the manufacture of coal tar shampoo in which the coal tar extract-detergent blend is neutralized sufficiently early in the cycle to permit substantially complete equilibration thereof before the formulation is released for packaging.

These and still further objects as shall hereinafter appear are readily fulfilled by the present invention in a remarkably unexpected manner as will be readily discerned from the following detailed description of an exemplary embodiment thereof.

The present invention is predicated upon the discovery of successful resolution of serious product problems inherent in prior art coal tar shampoos, namely, the inability of such products upon aging or the exposure to heat to retain its pleasing color and clarity, and viscosity standards characteristic of freshly

2

bottled product, and the ability to attain consistently a pre-selected pH during manufacture.

In attempting to resolve the prior art problems, it was learned that the sequence of introducing one or more of the several integral components of the shampoo is critical and that an equally critical relationship exists vis-a-vis the ratio of Fatty Acid DEA to CAPB.

Fatty acid diethanolamides have heretofore been suggested for use in shampoo formulations mainly as foam boosting and viscosity building agents. However, there was no suggestion in the prior art that a critical relationship might exist between the fatty acid diethanolamide and CAPB which could enhance shampoo stability. The level of fatty acid DEA, in accordance with this invention, will generally lie in the range of from 3% to 5% by weight based on the total weight of the composition. The level of CAPB will ordinarily be employed at concentrations in the range of from 1% to 4% on the same basis. The specific levels of each compound is to be selected such that the ratio fatty acid DEA/CAPB is within the range of 1.3:1 to 2.6:1.

The fatty acid DEA may be selected from those commercially available products where the fatty acid derivative is lauric, myristic, oleic, palmitic, stearic, tall oil or coconut. In the preferred form of this invention, optimum results are achieved by using the coconut fatty acid derivative or the lauric acid derivative.

One practice of the present invention wherein a coal tar shampoo is produced having all of the desired properties stated above while avoiding the costly and vexatious problems which plagued the prior art products will now be described. First, a homogenous particulate-free solution is prepared containing sodium lauryl ether sulfate ("SLES"), coal tar extract and purified water USP (hereinafter referred to as "Solution I").

Next, to be used as hereafter described, discrete solutions of anhydrous citric acid USP and sodium chloride are prepared (Solutions II and III, respectively).

Next a mixture of Fatty Acid DEA, methylparaben and propylparaben is blended together until the parabens are completely dissolved. Then the desired fragrance compound is added to the Fatty Acid DEA-paraben blend and thoroughly mixed therewith (Solution IV). This solution is covered and held until needed as will be later described.

To complete the manufacture of the new and improved coal-tar shampoo thereof, the coal tar extract-SLES solution (I) is placed in a batch mixing tank of adequate dimensions in which the mixer is running at moderate speed. Next the following ingredients are introduced one-at-a-time with continuous mixing to assure that the ingredient just added is totally dissolved before the subsequent ingredient is introduced: Tetrasodium EDTA; imidazolidinyl urea; citric acid solution (II); cocamidropropyl betaine ("CAPD"); and Fatty Acid DEA-paraben-fragrance solution (IV). Note that the ratio (by weight) of Fatty Acid DEA in Solution IV to CAPD must be between 1.3:1 and 2.6:1 if the full benefits of this invention are to be realized.

After producing the aforesaid mixture, the pH of the batch is measured using conventional techniques and such additional citric acid solution (Solution II) is added as is needed to bring the pH to 7.6. Finally, the viscosity is measured in cps using conventional techniques and sufficient sodium chloride solution (Solution III) is added qs to provide a reading of 8000 mPa.s. Both the citric acid and sodium chloride solutions are added using conventional techniques, i.e., only a portion, for instance 75%, of the theoretically required amount of reagent is added and mixed. Another reading is then taken and the procedure repeated until the desired value is reached. By following the procedure of adding only a portion of the stoichiometric required adjustment, the over running of the desired limit is avoided.

The batch is now ready for packaging into containers of any desired size using conventional sterile fill equipment.

To further illustrate the practice of the present invention, and not by way of limitation, the following examples are presented.

### Example I

A batch of coal tar shampoo is prepared to provide 3,785 liters of finished product. A base solution is prepared by introducing 1,495.5 kg of sodium lauryl ether sulfate to an adequately sized stainless steel mixing tank and, with medium agitation, 81.5 kg of coal tar extract is added thereto and mixed until homogeneous. 2,046.7 kg of purified water USP is thereafter introduced and agitation is continued until homogenity is obtained. The homogeneous solution is then filtered to remove unwanted particulate therefrom to provide 3,623.7 kg of Coal tar extract-SLES solution.

A citric acid solution is prepared in a separate stainless steel vessel of appropriate size by mixing equal 6.8 kg of anhydrous citric acid USP with 6.8 kg of purified water USP and solubilizing with a propeller mixer. This solution is then held until needed as hereinafter indicated.

A sodium chloride solution is prepared in a separate stainless steel vessel of appropriate size by mixing 25% by weight of sodium chloride and 75% by weight of purified water USP until all of the sodium chloride is dissolved and holding the resulting solution until needed as hereinafter indicated.

A solution of coconut fatty acid diethanolamide ("Coconut fatty acid DEA") is prepared by introducing 148.8 kg of coconut fatty acid DEA into an appropriately sized stainless steel container equipped with a mixer and thereafter adding 7.7 kg of methylparaben USP and 2.0 kg of propylparaben USP thereinto until the parabens are completely dissolved. Thereafter, 38.6 kg of a suitable fragrance compound is added thereto and mixed until it too is completely dissolved whereupon the vessel is covered and held for addition to the main batch as hereafter indicated.

The shampoo formulation is completed by placing the coal tar extract-SLES solution into the batch mixing tank. With the mixer running at moderate speed, the following ingredients are introduced discretely with dissolution or thorough mixing of the added ingredient being assured before the next ingredient is introduced. The first ingredient introduced into the 3,623.7 kg of coal tar extract-SLES solution is 1.2 kg of Tetrasodium EDTA (Technical grade). Thereafter, 19.3 kg of imidazolidinyl urea is added and thoroughly mixed. 7.0 kg of the citric acid solution is then added followed by 58.0 kg of cocamidopropyl Betaine. After the Betaine is thoroughly mixed into the batch, 197.0 kg of the coconut fatty acid DEA-paraben-fragrance solution is transferred from the covered vessel into the batch tank and it too is thoroughly mixed therewith.

Thereafter, the pH of the batch having attained reasonable equilibration, the pH is measured using conventional quality control techniques. Additional citric acid solution is then mixed therewith to the extent required to bring the batch pH to 7.6. Thereafter, an amount of sodium chloride solution is mixed qs into the batch in an amount sufficient to provide the batch with a viscosity of 8,000 mPa.s using standard measuring techniques. The batch is ready for packaging.

Example II

Several specimens of product produced according to Example I were compared with prior art product to determine stability of clarity when subjected to storage at 40°C (see Table A) and 50°C (see Table B). The specimens were checked periodically for haziness and clouding. The age of the specimen when haziness was first observed was recorded. As apparent from the data, samples prepared in accordance with the present invention endured a significantly longer period before exhibiting cloudiness. Thus, at 40°, the product of the present disclosure lasted an average of 4.7 months before clouding occurred in contrast to the 1.5 month average attained by the prior art product. At 50°C, product prepared by the present invention averaged 2.1 months before clouding whereas the prior art product averaged 1.4 months. The ability to retain clarity is an important attribute to the maintenance of cosmetic appeal and salable shelf life.

## TABLE A

### Clarity Stability After Storage at 40°C

| Lot. No. | Prior Art Method | Disclosure Method |
|---|---|---|
| 1001 | Two months | |
| 1002 | One month | |
| 1003 | One month | |
| 1004 | Two months | |
| 2001 | | Five months |
| 2002 | | Six months |
| 2003 | | Three months |
| 2004 | | Five months |
| 2005 | | Five months |
| 2006 | | Four months |
| 2007 | | Five months |
| Average | 1.5 months | 4.7 months |

*  *  *

## TABLE B

### Clarity Stability After Storage at 50°C

| Lot. No. | Prior Art Method | Disclosure Method |
|---|---|---|
| 1001 | One month | |
| 1002 | One month | |
| 1003 | One month | |
| 1004 | Two months | |
| 1005 | Two months | |
| 2001 | | Two months |
| 2002 | | Three months |
| 2003 | | Two months |
| 2004 | | Two months |
| 2005 | | Two months |
| 2006 | | Two months |
| 2007 | | Two months |
| Average | 1.4 months | 2.1 months |

### Example III

Several specimens of product produced according to the present disclosure were obtained for comparative color stability testing against a like number of specimens of prior art product. One half of the specimens were stored at 40°C (see Table C) and one half of the specimens were stored at 50°C (see Table D). Each specimen was measured at two week intervals to obtain Absorbance readings at 700 nm, the readings representing spectrophotometric measurements of darkness using standard measuring techniques, in which the larger numbers indicate a darker color.

As is apparent from the data, the prior art product darkened an average of 0.436 absorbance units after 12 weeks at 40°C and by an average of 1.282 units after 12 weeks at 50°C. Product of the present invention, on the other hand, darkened only 0.132 units after 12 weeks at 40°C and only 0.267 units after 12 weeks at 50°C.

## TABLE C

### Stability of Color Measurement after Storage at 40°C.

| | Prior Art Samples | | | Disclosure Samples | | |
|---|---|---|---|---|---|---|
| Period | 1001 | 1002 | 1005 | 2001 | 2003 | 2005 |
| Initial | 0.059 | 0.077 | 0.070 | 0.072 | 0.074 | 0.073 |
| 2 weeks | 0.094 | 0.135 | | | 0.152 | 0.106 |
| 4 weeks | 0.104 | 0.160 | 0.109 | 0.106 | | 0.123 |
| 6 weeks | 0.126 | 0.174 | | 0.134 | | 0.125 |
| 8 weeks | 0.166 | 0.193 | 0.155 | 0.146 | | 0.177 |
| 10 weeks | 0.202 | | | | | |
| 12 weeks | 0.216 | 0.139 | 1.023 | 0.189 | 0.227 | 0.198 |
| CHANGE | 0.216 | 0.139 | 0.953 | 0.117 | 0.153 | 0.125 |
| Average change | | 0.436 | | | 0.132 | |

## TABLE D

### Stability of Color Measurement after Storage at 50°C.

| | Prior Art Samples | | | Disclosure Samples | | |
|---|---|---|---|---|---|---|
| Period | 1001 | 1002 | 1005 | 2001 | 2003 | 2005 |
| Initial | 0.059 | 0.077 | 0.070 | 0.072 | 0.074 | 0.073 |
| 2 weeks | 0.117 | 0.179 | | 0.133 | 0.175 | 0.122 |
| 4 weeks | 0.146 | 0.193 | 0.164 | 0.145 | 0.231 | 0.143 |
| 6 weeks | 0.189 | 0.223 | | 0.263 | 0.242 | 0.195 |
| 8 weeks | 0.217 | 0.221 | 0.387 | 0.246 | 0.288 | 0.226 |
| 10 weeks | 0.352 | 1.058 | | 0.318 | 0.319 | 0.272 |
| 12 weeks | 0.781 | 1.679 | 1.594 | 0.337 | 0.356 | 0.327 |
| CHANGE | 0.722 | 1.602 | 1.524 | 0.265 | 0.282 | 0.254 |
| Average change | | 1.282 | | | 0.267 | |

### Example IV

Specimens of product prepared according to the present invention were compared with a like number of prior art specimens (i.e., product produced by prior methodology) to determine viscosity stability over an extended period of time. Product was stored at room temperature and viscosity was measured in cps using standard techniques for measuring viscosity. As shown in Table E, the prior art product lost from 2,930 mPa.s up to 5,129 mPa.s of viscosity over an eighteen month period whereas product produced according to the present disclosure not only did not lose any of its viscosity, it increased slightly instead.

## TABLE E

### Stability of Viscosity after Storage
### at room temperature
### (Viscosity measured at 25°C.)

| | Prior Art Samples | | | Disclosure Samples | | |
|---|---|---|---|---|---|---|
| Period | 1003 | 1001 | 1006 | 2004 | 2003 | 2001 |
| Initial | 6250 | 4350 | 6533 | 9100 | 8767 | 9232 |
| 4 months | 4165 | 3147 | 4900 | 9300 | 8200 | 11000 |
| 12 months | 1547 | 1520 | 3500 | 10300 | 11067 | 12967 |
| 18 months | 1130 | 1420 | 2500 | 9833 | 11200 | 13000 |
| CHANGE | -5129 | -2930 | -4033 | + 733 | +2433 | +3767 |

### Example V

Several batches of coal tar extract shampoo were prepared using both prior art methodology and the process of the present invention. Upon completion of formulation and prior to packaging, each batch was measured for pH using conventional methodology to determine its ease of replication. As shown in Table F, the prior art process obtained a standard deviation of 0.23 whereas the process of the present invention had a standard deviation of 0.10.

TABLE F

Initial pH Value After Manufacture

| Lot. No. | Prior Art Method | Disclosure Method |
|---|---|---|
| 1001 | 7.18 | |
| 1007 | 6.67 | |
| 1008 | 7.13 | |
| 1003 | 7.05 | |
| 1005 | 7.23 | |
| 1009 | 6.96 | |
| 1010 | 7.39 | |
| 2008 | | 7.66 |
| 2009 | | 7.71 |
| 2010 | | 7.50 |
| 2004 | | 7.47 |
| 2005 | | 7.67 |
| 2006 | | 7.69 |
| Standard Deviation | 0.23 | 0.10 |

Example VI

A coal tar extract shampoo was prepared using the method of Example I with solutions having the following composition:

Solution I

| | |
|---|---|
| Sodium Lauryl Ether Sulfate | 41.12% |
| Coal Tar Extract | 4.51% |
| POE (20) Sorbitan Monolaurate | 5.10% |
| Purified Water, USP | 49.27% |

Solution II

| | |
|---|---|
| Citric Acid | 50% |
| Purified Water, USP | 50% |

Solution III

| | |
|---|---|
| Sodium Chloride | 25% |
| Purified Water, USP | 75% |

Solution IV

| | |
|---|---|
| Lauric Acid DEA | 74.26% |
| Methylparaben, USP | 3.96% |
| Propylparaben, USP | 1.98% |
| Fragrance Compound | 19.80% |

Solution V

| | |
|---|---|
| Solution I | 88.27% |
| Solution II | 0.15% |
| EDTA | 0.03 |
| Imidazolidinyl Urea | 0.5% |
| Cocoamidopropyl Betaine | 2.85% |
| Sodium Lauryl Ether Sulfate (86%) | 3.0% |
| Solution IV | 5.05% |
| Solution III | qs |

The product produced thereby was tested and found to possess all the properties demonstrated in Examples II, III, IV, and V.

## Example VII

A coal tar extract shampoo was prepared using the method of Example I with solutions having the following composition:

**Solution I**

| | |
|---|---|
| Sodium Lauryl Ether Sulfate | 40.00% |
| Coal Tar Extracts | 2.00% |
| Purified Water, USP | 58.00% |

**Solution II**

| | |
|---|---|
| Citric Acid | 50% |
| Purified Water, USP | 50% |

**Solution III**

| | |
|---|---|
| Sodium Chloride | 25% |
| Purified Water, USP | 75% |

**Solution IV**

| | |
|---|---|
| Coconut Fatty Acid DEA | 80.25% |
| Methylparaben, USP | 4.93% |
| Propylparaben, USP | 2.47% |
| Fragrance Compound | 12.35% |

**Solution V**

| | |
|---|---|
| Solution I | 93.60% |
| Solution II | 0.15% |
| EDTA | 0.03 |
| Imidazolidinyl Urea | 0.5% |
| Cocoamidopropyl Betaine | 2.0% |
| Solution IV | 4.05% |
| Solution III | qs |

The product produced thereby was tested and found to possess all of the properties demonstrated in Examples II, III, IV, and V.

## Example VIII

A coal tar extract shampoo was prepared using the method of Example I with solutions having the following composition:

| Solution I | |
| --- | --- |
| Sodium Lauryl Ether Sulfate | 41.0% |
| Coal Tar Extract | 2.25 |
| Purified Water, USP | 56.75% |
| Solution II | |
| Citric Acid | 50% |
| Purified Water, USP | 50% |
| Solution III | |
| Sodium Chloride | 25% |
| Purified Water, USP | 75% |
| Solution IV | |
| Coconut Fatty Acid DEA | 86.20% |
| Methylparaben, USP | 3.45% |
| Propylparaben, USP | 1.72% |
| Fragrance Compound | 8.62% |
| Solution V | |
| Solution I | 91.2% |
| Solution II | 0.15% |
| EDTA | 0.03% |
| Imidazolidinyl Urea | 0.3% |
| Cocoamidopropyl Betaine | 2.5% |
| Solution IV | 5.8% |
| Solution III | qs |

The product produced thereby was tested and found to possess all of the properties demonstrated in Examples II, III, IV, and V.

Example IX

A coal tar extract shampoo was prepared using the method of Example I with solutions having the following composition:

| Solution I | |
|---|---|
| Sodium Lauryl Ether Sulfate | 41.2% |
| Coal Tar Extract | 2.25% |
| Purified Water, USP | 56.55% |
| Solution II | |
| Citric Acid | 50% |
| Purified Water, USP | 50% |
| Solution III | |
| Sodium Chloride | 25% |
| Purified Water, USP | 75% |
| Solution IV | |
| Lauric Acid DEA | 74.75% |
| Methylparaben, USP | 3.88% |
| Propylparaben, USP | 1.95% |
| Fragrance Compound | 19.42% |
| Solution V | |
| Solution I | 92.67% |
| Solution II | 0.15% |
| EDTA | 0.03 |
| Imidazolidinyl Urea | 0.5% |
| Cocoamidopropyl Betaine | 1.5% |
| Solution IV | 5.15% |
| Solution III | qs |

The product produced thereby was tested and found to possess all of the properties demonstrated in Examples II, III, IV, and V.

Example X

A coal tar extract shampoo was prepared using the method of Example I with solutions having the following composition:

Solution I

| | |
|---|---|
| Sodium Lauryl Ether Sulfate | 48.0% |
| Coal Tar Extract | 2.25% |
| Purified Water, USP | 49.75% |

Solution II

| | |
|---|---|
| Citric Acid | 50% |
| Purified Water, USP | 50% |

Solution III

| | |
|---|---|
| Sodium Chloride | 25% |
| Purified Water, USP | 75% |

Solution IV

| | |
|---|---|
| Lauric Acid DEA | 69.76% |
| Methylparaben, USP | 4.66% |
| Propylparaben, USP | 2.33% |
| Fragrance Compound | 23.25% |

Solution V

| | |
|---|---|
| Solution I | 86.0% |
| Solution II | 0.15% |
| EDTA | 0.03 |
| Imidazolidinyl Urea | 0.5% |
| Cocoamidopropyl Betaine | 1.25% |
| Solution IV | 4.30% |
| Solution III | qs |
| Purified Water, USP | 7.75% |

The product produced thereby was tested and found to possess all of the properties demonstrated in Examples II, III, IV, and V.

Example XI

A coal tar extract shampoo was prepared using the method of Example I with solutions having the following composition:

Solution I

| | | |
|---|---|---|
| Sodium Lauryl Ether Sulfate | 41.27% |
| Coal Tar Extract | 2.25% |
| Purified Water, USP | 49.75% |

Solution II

| | |
|---|---|
| Citric Acid | 50% |
| Purified Water, USP | 50% |

Solution III

| | |
|---|---|
| Sodium Chloride | 25% |
| Purified Water, USP | 75% |

Solution IV

| | |
|---|---|
| Coconut Fatty Acid DEA | 74.75% |
| Methylparaben, USP | 3.88% |
| Propylparaben, USP | 1.95% |
| Fragrance Compound | 19.42% |

Solution V

| | |
|---|---|
| Solution I | 92.77% |
| Solution II | 0.15% |
| EDTA | 0.03 |
| Imidazolidinyl Urea | 0.5% |
| Cocoamidopropyl Betaine | 1.5% |
| Solution IV | 5.15% |
| Solution III | qs |

The product produced thereby was tested and found to possess all of the properties demonstrated in Examples II, III, IV, and V.

From the foregoing, it is apparent that methods and compositions have been herein described which fulfill all of the aforestated objectives in a remarkably unexpected fashion. It is of course understood that such modifications, alterations and adaptations as may readily occur to the artisan confronted with this disclosure are intended within the spirit of this disclosure which is limited only by the scope of the claims appended hereto.

**Claims**

1. The method of producing a coal tar-containing shampoo having stable viscosity, color and clarity comprising:

premixing a plurality of descrete solutions respectively containing in weight percent

(a) from 40 to 48 percent particulate free sodium lauryl ether sulfate ("SLES"), from 2 to 5 percent coal tar extract and from 47 to 58 percent purified water USP, (herein "Solution I");

(b) 50% anhydrous citric acid USP and 50% purified water USP, (herein "Solution II');

(c) 25% sodium chloride and 75% purified water, USP, (herein "Solution III");

(d) 69—81% fatty acid DEA, 4.9 to 7.4% paraben selected from methylparaben, propylparaben and mixtures thereof, and from 8 to 24 per cent fragrance (herein "Solution IV");

introducing by weight percent of the final formulation, from 88 to 94 percent of Solution I into a stainless steel mixing tank with moderate mixing; adding 0.03 percent of tetrasodium EDTA into Solution I and mixing it thoroughly therein to form a first mixture therewith; thereafter introducing from 0.3 to 0.5 percent imidazolidinyl urea into said first mixture and mixing thoroughly to form a second mixture; adding from 0.1 to 0.2 percent of Solution II into said second mixture to form a third mixture; admixing from 1.2 to 2.9 percent cocoamidopropl betaine ("CAPB") into said third mixture to form a fourth mixture having said CAPB thoroughly mixed therethrough and providing said mixture with a Fatty acid DEA :: CAPB ratio of at least 1.3:1 and not more than 2.6:1, thereafter admixing from 4.0 to 5.8 percent of Solution IV into and throughout said fourth mixture to form a fifth mixture; measuring the pH of said fifth mixture; adjusting the pH of said fifth mixture to 7.6; and packaging said pH adjusted fifth mixture.

2. A process according to claim 1 in which a sufficient amount of Solution II is admixed into said fourth mixture to provide a pH = 7.6.

3. A process according to claim 1 in which a sufficient amount of Solution III is admixed into said pH adjusted fifth mixture to provide said batch with a viscosity of 8000 mpa.s.

4. A process according to claim 1 in which said Fatty acid DEA is selected from lauric, myristic, oleic, palmitic, stearic, tall oil or coconut.

5. A process according to claim 2 in which said Fatty acid DEA is selected from lauric, myristic, oleic, palmitic, stearic, tall oil or coconut.

6. A process according to claim 3 in which said Fatty acid DEA is selected from lauric, myristic, oleic, plamitic, stearic, tall oil or coconut.

7. A process according to claim 1 in which said Fatty acid DEA is cocodiethanolamide.

8. A coal tar containing shampoo having stable viscosity, color and clarity on standing consisting, in weight percent of:

| | |
|---|---|
| Sodium lauryl ether sulfate | 35—45 |
| Coal tar extract | 1.75—4.7 |
| Anhydrous citric acid | .05—0.1 |
| Fatty acid DEA | 3—5 |
| Methylparaben, USP | 0.8—3.2 |
| Propylparaben, USP | 0.2—0.8 |
| EDTA | 0.03 |
| Imidazolidinyl urea | 0.3—0.5 |
| Cocoamidopropyl betaine (CAPB) | 1—4 |
| Purified Water, USP | 36.67—57.65 |
| Fragrance compound | 0.32—1.4 |

in which the ratio of fatty acid DEA to CAPB is at least 1.3:1 and not more than 2.6:1.

9. A coal tar containing shampoo according to claim 8 in which said Fatty acid DEA is selected from lauric, myristic, oleic, palmitic, stearic, tall oil or coconut.

10. A coal tar containing shampoo according to claim 9 in which said Fatty acid DEA is cocodiethanolamide.

11. A coal tar containing shampoo according to claim 8, which further includes sodium chloride.

**Patentansprüche**

1. Verfahren zur Herstellung eines kohlenteerhaltigen Shampoos mit beständiger Viskosität, Farbe und Klarheit, umfassend

Vormischen einer Vielzahl von einzelnen Lösungen, die in Gewichtsprozent jeweils enthalten:

(a) 40—48% teilchenfreies Natriumlaurylethersulfat ("SLES"), 2—5% Kohlenteerextrakt und 47—58% gereinigtes Wasser nach USP (hier "Lösung I");

(b) 50% wasserfreie Zitronensäure nach USP und 50% gereinigtes Wasser nach USP (hier "Lösung II");

(c) 25% Natriumchlorid und 75% gereinigtes Wasser nach USP (hier "Lösung III");

(d) 69—81% Fettsäure-DEA, 4,9—7,4% Paraben, ausgewählt aus Methylparaben, Propylparaben und Gemischen davon, und 8—24% Duftstoffe (hier "Lösung IV");

Einbringen — in Gewichtsprozent der Endformulierung — 88—94% von Lösung I in einen Mischtank aus rostfreiem Stahl unter mäßigem Vermischen; Zugeben von 0,03% Tetranatrium-EDTA in Lösung I und gründliches Vermischen darin unter Bildung eines ersten Gemischs damit; anschließendes Einbringen von 0,3—0,5% Imidazolidinylharnstoff in das erste Gemisch und gründliches Vermischen unter Bildung eines zweiten Gemischs; Zugeben von 0,1—0,2% von Lösung II in das zweite Gemisch unter Bildung eines dritten Gemischs; Beimischen von 1,2—2,9% Cocamidopropylbetain ("CAPB") in das dritte Gemisch unter Bildung eines vierten Gemischs, das mit dem CAPB gründlich durchmischt ist, so daß das Gemisch ein Verhältnis von Fettsäure-DEA:CAPB von wenigstens 1,3:1 und höchstens 2,6:1 hat; anschließendes Beimischen von 4,0—5,8% von Lösung IV in und durch das gesamte vierte Gemisch unter Bildung eines fünften Gemischs; Messen des pH-Werts des fünten Gemischs; Einstellen des pH-Werts des fünften Gemischs auf 7,6; und Verpacken des fünften Gemischs mit eingestelltem pH-Wert.

2. Verfahren nach Anspruch 1, wobei zur Einstellung eines pH-Werts von 7,6 dem vierten Gemisch eine ausreichende Menge von Lösung II beigemischt wird.

3. Verfahren nach Anspruch 1, wobei dem fünften Gemisch mit eingestelltem pH-Wert eine ausreichende Menge von Lösung III beigemischt wird, um der Charge eine Viskosität von 8000 mPa·s zu geben.

4. Verfahren nach Anspruch 1, wobei das Fettsäure-DEA aus Laurin-, Myristin-, Olein-, Palmitin-, Stearinsäure, Tallöl oder Kokosnuß ausgewählt wird.

5. Verfahren nach Anspruch 2, wobei das Fettsäure-DEA aus Laurin-, Myristin-, Olein-, Palmitin-, Stearinsäure, Tallöl oder Kokosnuß ausgewählt wird.

6. Verfahren nach Anspruch 3, wobei das Fettsäure-DEA aus Laurin-, Myristin-, Olein-, Palmitin-, Stearinsäure, Tallöl oder Kokosnuß ausgewählt wird.

7. Verfahren nach Anspruch 1, wobei das Fettsäure-DEA Cocodiethanolamid ist.

8. Kohlenteerhaltiges Shampoo mit beständiger viskosität, Farbe und Klarheit bei Lagerung, das in Gewichtsprozent besteht aus:

| | |
|---|---|
| Natriumlaurylethersulfat | 35—45 |
| Kohlenteerextrakt | 1,75—4,7 |
| wasserfreier Zitronensäure | 0,05—0,1 |
| Fettsäure-DEA | 3—5 |
| Methylparaben, USP | 0,8—3,2 |
| Propylparaben, USP | 0,2—0,8 |
| EDTA | 0,03 |
| Imidazolidinylharnstoff | 0,3—0,5 |
| Cocoamidopropylbetain (CAPB) | 1—4 |
| gereinigtem Wasser, USP | 36,67—57,65 |
| Duftstoffverbindung | 0,32—1,4 |

wobei das Verhältnis von Fettsäure-DEA zu CAPB wenigstens 1,3:1 und höchstens 2,6:1 beträgt.

9. Kohlenteerhaltiges Shampoo nach Anspruch 8, wobei das Fettsäure-DEA aus Laurin-, Myristin-, Olein-, Palmitin-, Stearinsäure, Tallöl oder Kokosnuß ausgewählt ist.

10. Kohlenteerhaltiges Shampoo nach Anspruch 9, wobei das Fettsäure-DEA Cocodiethanolamid ist.

11. Kohlenteerhaltiges Shampoo nach Anspruch 8, das ferner Natriumchlorid enthält.

**Revendications**

1. Procédé de préparation d'un shampooing contenant du coaltar, ayant une viscosité, une couleur et une limpidité stables, comprenant les opérations consistant:

à prémélanger plusieurs solutions séparées, contenant respectivement, en pourcentages en poids

(a) de 40 à 48% de sulfate de lauryléther de sodium ("SLES") exempt de particules, de 2 à 5% d'extrait de coaltar et de 47 à 58% d'eau purifiée USP (= aux termes de la pharmacopée des Etats-Unis) ("solution I"),

(b) 50% d'acide citrique anhydre USP et 50% d'eau purifiée USP ("solution II"),

(c) 25% de chlorure de sodium et 75% d'eau purifié USP ("solution III"),

(d) de 69 à 81% de DEA (= diéthanolamide) d'acide gras, de 4,9 à 7,4% d'un parahydroxybenzoate choisi parmi le parahydroxybenzoate de méthyle, le parahydroxybenzoate de propyl et des mélanges de ceux-ci, et de 8 à 24% de parfum ("solution IV");

à introduire, en pourcentages en poids de la préparation finale, de 88 à 94% de la solution I dans une cuve-mélangeur en acier inoxydable à mélange modéré; à adjouter 0,03% d'EDTA tétrasodique à la solution I et a l'y mélanger intimement pour former un premier mélange; puis à introduire de 0,3 à 0,5% d'imidazolidinyl-urée dans ledit premier mélange et à mélanger intimement pour former un deuxième mélange; a ajouter de 0,1 à 0,2% de solution II audit deuxième mélange pour former un troisième mélange; à ajouter de 1,2 à 2,9% de cocamidopropyl-bétaïne ("CAPB") audit troisième mélange pour former un quatrième mélange dans lequel ladite CAPB est mélangée intimement, le rapport DEA d'acide gras/CAPB de ce mélange étant de 1,3:1 au moins et de 2,6:1 au plus; puis à mélanger de 4,0 à 5,8% de solution IV audit quatrième mélange pour former un cinquième mélange; à mesurer le pH dudit cinquième mélange; à régler le pH dudit cinquième mélange à 7,6; et à emballer ledit cinquième mélange dont le pH est réglé.

2. Procédé selon la revendication 1, dans lequel on ajoute, audit quatrième mélange, une quantité suffisante de solution II pour lui donner un pH = 7.6.

3. Procédé selon la revendication 1, dans lequel on ajoute, audit cinquième mélange dont le pH est réglé, une quantité suffisante de solution III pour donner à la charge une viscosité de 8000 mPa.s.

4. Procédé selon la revendication 1, dans lequel ledit DEA d'acide gras est choisi parmi les DEA laurique, myristique, oléique, palmitique, stéarique, de tallol ou d'huile de coco.

5. Procédé selon la revendication 2, dans lequel ledit DEA d'acide gras est choisi parmi les DEA laurique, myristique, oléique, palmitique, stéarique, de tallol ou d'huile de coco.

6. Procédé selon la revendication 3, dans lequel ledit DEA d'acide gras est choisi parmi les DEA laurique, myristique, oléique, palmitique, stéarique, de tallol ou d'huile de coco.

7. Procédé selon la revendication 1, dans lequel ledit DEA d'acide gras est le diéthanolamide d'huile de coco.

8. Shampooing contenant du coaltar, de viscosité, de couleur et de limpidité stables au stockage, ayant la composition suivante en pourcentages en poids:

| | |
|---|---|
| Sulfate de lauryléther de sodium | 35—45 |
| Extrait de coaltar | 1,75—4,7 |
| Acide citrique anhydre | 0,05—0,1 |
| DEA d'acide gras | 3—5 |
| Parahydroxybenzoate de méthyle, USP | 0,8—3,2 |
| Parahydroxybenzoate de propyle, USP | 0,2—0,8 |
| EDTA | 0,03 |
| Imidazolidinyl-urée | 0,3—0,5 |
| Cocoamidopropyl-bétaïne (CAPB) | 1—4 |
| Eau purifiée, USP | 36,67—57,65 |
| Parfum | 0,32—1,4 |

le rapport du DEA d'acide gras à la CAPB étant de 1,3:1 au moins et de 2,6:1 au plus.

9. Shampooing contenant du coaltar selon la revendication 8, dans lequel ledit DEA d'acide gras est choisi parmi les DEA laurique, myristique, oléique, palmitique, stéarique, de tallol ou d'huile de coco.

10. Shampooing contenant du coaltar selon la revendication 9, dans lequel ledit DEA d'acide gras est le diéthanolamide d'huile de coco.

11. Shampooing contenant du coaltar selon la revendication 8, contenant en outre du chlorure de sodium.